# EUROPÄISCHE PATENTSCHRIFT

(11) **EP 0 547 465 B1**
(45) Veröffentlichungstag und Bekanntmachung des Hinweises auf die Patenterteilung: **07.05.1997**
(21) Anmeldenummer: 92120843.5
(22) Anmeldetag: 07.12.1992
(51) Int. Cl.: A61K 35/78

(54) **Verfahren zur Herstellung von Extrakten aus Rhizoma Petasitidis**
Process of preparation of extracts of Petasites rhizomas
Procédé de préparation d'extraits de rhizome de Petasites

(30) Priorität: 14.12.1991 DE 4141749
(43) Veröffentlichungstag der Anmeldung: 23.06.1993
(73) Patentinhaber: WEBER & WEBER GmbH, D-82263 Inning (DE)
(72) Erfinder: Koch, Volkmar, Dr., W-8084 Inning/Buch (DE); Höcherl, Siegfried, Dipl.-Chem, W-8911 Schwifting (DE)
(74) Vertreter: Winkler, Andreas, Dr.

(56) Entgegenhaltungen:
- EP-A- 0 508 330
- DE-A- 4 002 938
- DE-C- 897 145

## Beschreibung

Die Erfindung betrifft ein Verfahren zur Herstellung von Extrakten aus Rhizoma Petasitidis mit stark reduziertem Pyrrolizidinalkaloidgehalt durch Hochdruckextraktion mit Kohlendioxid.

Pestwurz, Petasites hybridus oder Petasites officinalis [L.] Moendi (Astralis), wurde bereits in der Volksheilkunde wegen ihrer schleimlösenden Eigenschaften bei Husten und Asthma sowie wegen ihrer spasmolytischen Wirkung bei Krampfzuständen und Darmspasmen therapeutisch eingesetzt. In heutigen pharmazeutischen Präparaten kommen insbesondere Extrakte aus Rhizoma Petasitidis, d.h. Extrakte aus dem Wurzelstock der Pestwurz, zum Einsatz. In jüngster Vergangenheit hat sich der Indikationsbereich derartiger Extrakte noch erheblich ausgeweitet. So beschreibt beispielsweise die europäische Patentanmeldung Nr. 87 103 561.4 als zusätzliche Indikationen verschiedenartige gastrointestinale Erkrankungen.

Als Hauptwirkstoff der aus Rhizoma Petasitidis extrahierbaren Substanzen ist das Petasin anzusehen, ein Ester aus Petasol und Angelicasäure. Daneben sind in dieser Arzneimittelpflanze eine Reihe von Pyrrolizidinalkaloiden nachgewiesen worden. Das hepatotoxische und kanzerogene Potential dieser Pyrrolizidinalkaloide hat in der Öffentlichkeit kontrovers geführte Diskussionen über die Verwendung des Extraktes als Arzneimittel ausgelöst. In jedem Fall ist es aus Gründen der Vorsorge wünschenswert, den Gehalt an Pyrrolizidinalkaloiden in Extrakten aus Rhizoma Petasitidis so gering wie möglich zu halten.

Zur Lösung dieses Problems sind bisher zwei Verfahren bekannt geworden. Gemäß einem ersten Verfahren (Ch. Mauz et al., Pharmazeutica Acta Helvetiae, Bd. 60 (1985), S. 256-259) wird ein alkoholischer Extrakt aus Rhizoma Petasitidis mit einem Kationenaustauscher behandelt. Auf diese Weise werden mehr als 93% der Pyrrolizidinalkaloide am Ionenaustauscher adsorbiert. Dieses Verfahren ist ziemlich aufwendig, weil der Extrakt anschließend von Ionenaustauscher, Alkohol und Wasser abgetrennt werden muß, was im Falle von Alkohol und Wasser wegen der Temperaturempfindlichkeit der Pflanzeninhaltstoffe nicht unproblematisch ist.

Aus der DE-PS 39 10 831 ist ein Verfahren für die Herstellung von Extrakten aus Rhizoma Petasitidis mit stark reduziertem Pyrrolizidinalkaloidgehalt durch Hochdruckextraktion mit Kohlendioxid bekannt. Mit diesem Verfahren ist es möglich, Extrakte zu erhalten, deren Gehalt an Pyrrolizidinalkaloiden unter 5 ppm liegt. Ein sicheres Absenken der Pyrrolizidinalkaloide unter die Nachweisgrenze von 0,1 ppm ist nach diesem Verfahren allerdings nicht in jedem Falle möglich.

Besondere Schwierigkeiten ergeben sich, wenn bei der Extraktion oder einer Nachbehandlung anfallendes Wasser aus der Droge oder aus einer separaten Wasserzugabe aus dem Extrakt entzogen werden muß, um ein klares, verkaufsfähiges Produkt zu erhalten. Üblicherweise wird das Wasser zunächst durch Abschöpfung und/oder Zentrifugation bis auf etwa 10 Gew.-% abgetrennt. Eine weitere Wasserabtrennung aus dem Extrakt ist aufgrund der Dichte des Extraktes, die nahe bei 1 liegt, problematisch. Daher werden die restlichen 10% emulgiertes Wasser üblicherweise mit Natriumsulfat oder einem ähnlichen Trockenmittel gebunden. Wie neuere Untersuchungen ergeben haben, scheinen bei diesem Vorgang voraussichtlich noch im Wasser gelöste Pyrrolizidinalkaloide in den Extrakt überzutreten, so daß deren Konzentration im Extrakt durch den abschließenden Trocknungsschritt wieder ansteigt.

Aus EP-A-0 508 330 (Stand der Technik nach Artikel 54(3) EPÜ), ist ein Verfahren zur Herstellung von Extrakten aus Rhizoma Petasitidis durch Extraktion der Droge mit verdichtetem Kohlendioxid bekannt, bei dem zur Weiterbehandlung des Extraktes der Weg eingeschlagen wird, daß der Extrakt zunächst mit der 1-10-fachen Menge an Wasser behandelt wird. Nach der Entmischung der Phasen wird die Extraktphase abgetrennt und getrocknet, wobei die Trocknung über einem Trockenmittel, wie Natriumsulfat, erfolgt.

Aus der DE 40 02 938 ist ein weiteres gattungsgemäßes Verfahren bekannt, bei dem der Restwassergehalt im Extrakt durch Trocknen des Extraktes oder durch Filtration oder durch Zentrifugation über einem Trocknungsmittel durchgeführt wird.

Der Erfindung liegt daher die Aufgabe zugrunde, das bekannte Verfahren dahingehend weiterzuentwickeln, daß der Pyrrolizidinalkaloidgehalt im verkaufsfertigen Extrakt aus Rhizoma Petasitidis zuverlässig und auf einfache Weise unter die Nachweisgrenze abgesenkt wird.

Erfindungsgemäß wird diese Aufgabe dadurch gelöst, daß bei und/oder nach der Extraktion anfallendes Wasser im wesentlichen ausschließlich mit Hilfe einer Durchlaufzentrifugation bis zu einem Wassergehalt von unter 1 Gew.-% abgetrennt wird.

In einer bevorzugten Ausführungsform der Erfindung ist vorgesehen, daß im Anschluß an die Durchlaufzentrifugation der verbleibende Extrakt mindestens einmal mit der ein- bis zehnfachen Menge an Säure ausgeschüttelt, die Säurephase abgetrennt, der Extrakt durch mehrmaliges Waschen auf einen pH-Wert von 3-7 gebracht und der Extrakt abschließend wieder auf einen Wassergehalt von unter 1 Gew.-% gebracht wird.

Dabei wird vorgeschlagen, daß als Säure eine Mineralsäure, vorzugsweise Schwefelsäure, verwendet wird.

Weiterhin sieht die Erfindung vor, daß die Säure eine Normalität von 0,1 bis 1 besitzt.

In einer bevorzugten Ausführungsform der Erfindung wird vorgeschlagen, daß das Ausschütteln mehrfach wiederholt wird.

Besonders bevorzugt wird der Extrakt mit der ein- bis zweifachen Menge an Säure ausgeschüttelt wird.

Weiterhin sieht die Erfindung vor, daß die Abtrennung der Säure- bzw. Wasserphasen mit Hilfe der Durchlaufzentrifugation erfolgt.

Erfindungsgemäß ist vorgesehen, daß die Bindung des Restwassergehaltes mit Hilfe eines Trockenmittels wie z.B. Natriumsulfat erfolgt.

Völlig überraschend ist es mit Hilfe der Durchlaufzentrifugation gelungen, das im Extrakt emulgierte Wasser ohne den Einsatz von Trockenmitteln auf unter 1 Gew.-% abzusenken und damit, verkaufsfertige Extrakte zu erhalten, in denen die Pyrrolizidinalkaloide auch mit dem empfindlichen und hochselektiven Methoden der GC-MS-Kopplung nicht mehr nachgewiesen werden können. Die Nachweisgrenze dieser Analyseverfahren liegt bei etwa 0,1 ppm, so daß man von einem Gehalt von in jedem Falle unter 0,1 ppm ausgehen kann.

Die Verfahrensbedingungen der Hochdruckextraktion mit Kohlendioxid entsprechen im wesentlichen den in der DE-PS 39 10 831 angegebenen Werten, d.h. die Extraktion wird bei einem Druck von 100 bis 400 bar, vorzugsweise 130 bis 250 bar; einer Temperatur zwischen 25 und 45°C, vorzugsweise bei ca. 35°C; einem Kohlendioxid-Durchsatz von 200 bis 500 kg/h (was einem Durchsatz von etwa 5 bis 50 kg CO₂ pro kg Droge entspricht) und einer Extraktionsdauer von 100 bis 200 min durchgeführt.

Dieser mit dem sogenannten CO₂-Verfahren gewonnene Extrakt wird nach Abschöpfen des überstehenden Wasser mit Hilfe der Durchlaufzentrifugation auf unter 1 Gew.-% Wasser getrocknet.

Durch die unerwartet effektive Trocknung mit Hilfe der Durchlaufzentrifugation konnten Drogen mit einem Wassergehalt von bis zu 15 Gew.-% eingesetzt werden, wobei sich bei ca. 10 Gew.-% Wassergehalt eine stabile Emulsion bildet. Die Ausbeute der Extraktion lag bei etwa 3 Gew.-% (bezogen auf die eingesetzte Droge) eines klaren, lipophilen, bei Zimmertemperatur zähflüssigen Extraktes mit einem Petasingehalt von bis zu 40 Gew.-% und einem Wassergehalt von 0,5 bis 1 Gew.-%.

Obgleich bereits die Durchlaufzentrifugation allein befriedigende Ergebnisse liefert, wird in einer bevorzugten Ausführungsform der Erfindung aus Sicherheitsgründen eine Ausschüttelung mit 0,1 bis 1,0 N Mineralsäure, bspw. 0,1 N Schwefelsäure, durchgeführt, wobei auf 1 Teil Extrakt vorzugsweise 2 Teile Säure kommen. Durch die Ansäuerung der wäßrigen Phase durch H₂SO₄ wird wohl eine weitgehende Salzbildung der freien Alkaloide erreicht, die im neutralen, wäßrigen Millieu nicht möglich ist. Hierdurch können vermutlich die Pyrrolizidinalkaloide in noch stärkerem Maße von der lipophilen Phase in die wäßrige Phase übergehen. Die abschließende Abtrennung der sauren Phase(n) durch vorzugsweise Durchlaufzentrifugation (vorzugsweise bei über 40.000 G) ermöglicht dann eine Entfernung der in die saure Phase übergegangenen Alkaloide. Anschließend muß der Extrakt noch auf einen pH von 3-7, bspw. 5,5, gewaschen werden und im letzten Schritt wieder per Durchlaufzentrifugation auf einen Wert von unter 1 Gew.-% Wassergehalt gebracht werden. Abschließend kann dann noch eine Bindung des Restwassergehaltes mit Hilfe eines Trockenmittels erfolgen, da nunmehr die Pyrrolizidinalkaloide soweit abgereichert sind und der Wassergehalt so verringert ist, daß die anfangs beschriebenen Nachteile nicht mehr auftreten.

Für alle Durchlaufzentrifugationen wurde eine Zentrifuge der Firma Padberg verwendet. Eine Absenkung des Wassergehalts auf 1 Gew.-% war mit diesem Gerät bei über 40.000 G möglich. Selbstverständlich können bei nach demselben Prinzip arbeitenden Geräten anderer Firmen andere Umdrehungszahlen für die Zentrifugation angemessener sein

## Patentansprüche

1. Verfahren zur Herstellung von Extrakten aus Rhizoma Petasitidis mit stark reduziertem Pyrrolizidinalkaloidgehalt durch Hochdruckextraktion mit Kohlendioxid,
dadurch gekennzeichnet,
daß bei und/oder nach der Extraktion anfallendes Wasser im wesentlichen ausschließlich mit Hilfe einer Durchlaufzentrifugation bis zu einem Wassergehalt von unter 1 Gew.-% abgetrennt wird.

2. Verfahren nach Anspruch 1, dadurch gekennzeichnet, daß im Anschluß an die Durchlaufzentrifugation der verbleibende Extrakt mindestens einmal mit der ein- bis zehnfachen Menge an Säure ausgeschüttelt, die Säurephase abgetrennt, der Extrakt durch Waschen auf einen pH-Wert von 3-7 gebracht und der Extrakt abschließend wieder auf einen Wassergehalt von unter 1 Gew.-% gebracht wird.

3. Verfahren nach Anspruch 2, dadurch gekennzeichnet, daß als Säure eine Mineralsäure verwendet wird.

4. Verfahren nach Anspruch 3, dadurch gekennzeichnet, daß als Säure Schwefelsäure verwendet wird.

5. Verfahren nach einem der Ansprüche 2 bis 4, dadurch gekennzeichnet, daß die Säure eine Normalität von 0,1 bis 1 besitzt.

6. Verfahren nach einem der Ansprüche 2 bis 5, dadurch gekennzeichnet, daß das Ausschütteln mehrfach wiederholt wird.

7. Verfahren nach einem der Ansprüche 2 bis 6, dadurch gekennzeichnet, daß der Extrakt mit der ein- bis zweifachen Menge an Säure ausgeschüttelt wird.

8. Verfahren nach einem der Ansprüche 2 bis 7, dadurch gekennzeichnet, daß die Abtrennung der Säure- bzw. Wasserphasen mit Hilfe der Durchlaufzentrifugation erfolgt.

9. Verfahren nach einem der vorangehenden Ansprüche, dadurch gekennzeichnet, daß die Bindung des Restwassergehaltes mit Hilfe eines Trockenmittels, wie z.B. Natriumsulfat, erfolgt.

## Claims

1. A method of preparing extracts of rhizoma petasitidis with a greatly reduced pyrrolizidine alkaloid content by high-pressure extraction with carbon dioxide,
characterised in that
water appearing during and/or after extraction is removed, substantially exclusively by continuous centrifuging, down to a water content of below 1% by weight.

2. A method according to claim 1, characterised in that after continuous centrifuging, the remaining extract is shaken out at least once with one to ten times the amount of acid, the acid phase is separated, the extract is brought to a pH of 3 - 7 by washing, and the extract is finally brought back to a water content of below 1% by weight.

3. A method according to claim 2, characterised in that the acid used is a mineral acid.

4. A method according to claim 3, characterised in that the acid used is sulphuric acid.

5. A method according to any of claims 2 to 4, characterised in that the acid has a strength of 0.1 N to 1 N.

6. A method according to any of claims 2 to 5, characterised in that the shaking-out process is repeated more than once.

7. A method according to one of claims 2 to 6, characterised in that the extract is shaken out with one to two times the amount of acid.

8. A method according to any of claims 2 to 7, characterised in that the acid and/or water phases are separated by continuous centrifuging.

9. A method according to any of the preceding claims, characterised in that the remaining water content is bonded by a drying agent such as sodium sulphate.

## Revendications

1. Procédé de fabrication d'extraits de Rhizoma Petasitidis d'une teneur fortement réduite en alcaloïde de pyrrolizidine, par extraction sous pression élevée avec du dioxyde de carbone,
caractérisé
par le fait que, concernant l'eau produite lors et/ou après extraction, on procède à sa séparation pratiquement exclusivement à l'aide d'une centrifugation en continu, jusqu'à atteindre une teneur en eau inférieure à 1 % en poids.

2. Procédé selon la revendication 1, caractérisé par le fait que, suite à la centrifugation en continu, l'extrait subsistant est expulsé au moins une première fois avec une quantité d'acide allant du simple au décuple, la phase acide étant séparée, l'extrait étant amené à un pH compris dans la plage allant de 3 à 7, par lavage, et l'extrait étant ensuite remis à une teneur en eau inférieure à 1 % en poids.

3. Procédé selon la revendication 2, caractérisé par le fait que l'on utilise comme acide un acide minéral.

4. Procédé selon la revendication 3, caractérisé par le fait que l'on utilise comme acide de l'acide sulfurique.

5. Procédé selon l'une des revendications 2 à 4, caractérisé par le fait que l'acide a un titrage de normalité de 0,1 à 1.

6. Procédé selon l'une des revendications 2 à 5, caractérisé par le fait que l'extraction par solvant est répétée plusieurs fois.

7. Procédé selon l'une des revendications 2 à 6, caractérisé par le fait que l'extrait est agité avec une quantité d'acide allant du simple au décuple.

8. Procédé selon l'une des revendications 2 à 7, caractérisé par le fait que la séparation des phases acide, respectivement aqueuse, est éffectuée à l'aide d'une centrifugation continue.

9. Procédé selon l'une des revendications précédentes, caractérisé par le fait que la condition imposée à la teneur en eau résiduelle s'obtient à l'aide d'un agent dessiccant, tel que par exemple du sulfate de sodium.
